(19) Europäisches Patentamt
European Patent Office
Office européen des brevets

(11) **EP 1 524 935 B1**

(12) **EUROPÄISCHE PATENTSCHRIFT**

(45) Veröffentlichungstag und Bekanntmachung des Hinweises auf die Patenterteilung:
**07.10.2009 Patentblatt 2009/41**

(21) Anmeldenummer: **03787631.5**

(22) Anmeldetag: **05.07.2003**

(51) Int Cl.:
*A61B 5/0484* (2006.01)    *A61M 21/00* (2006.01)

(86) Internationale Anmeldenummer:
**PCT/DE2003/002250**

(87) Internationale Veröffentlichungsnummer:
**WO 2004/016165 (26.02.2004 Gazette 2004/09)**

(54) **Medizinische Vorrichtung zur Modulation neuronaler Aktivität im Gehirn**

Medical device for modulation of neuronal activity in the brain

Dispositif médical pour moduler une activité neuronale dans le cerveau

(84) Benannte Vertragsstaaten:
**AT BE BG CH CY CZ DE DK EE ES FI FR GB GR HU IE IT LI LU MC NL PT RO SE SI SK TR**

(30) Priorität: **29.07.2002 DE 10233960**

(43) Veröffentlichungstag der Anmeldung:
**27.04.2005 Patentblatt 2005/17**

(73) Patentinhaber: **Forschungszentrum Jülich GmbH 52425 Jülich (DE)**

(72) Erfinder: **TASS, Peter 52445 Titz (DE)**

(74) Vertreter: **Patentanwälte Lambsdorff & Lange Dingolfinger Strasse 6 81673 München (DE)**

(56) Entgegenhaltungen:
US-A- 3 780 724        US-A- 3 892 227
US-A- 4 171 696        US-A- 4 201 224
US-A- 4 417 592        US-A- 5 540 235

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 524 935 B1

**Beschreibung**

Die Erfindung betrifft eine Vorrichtung zur bedarfsgesteuerten Modulation physiologischer und pathologischer neuronaler rhythmischer Aktivität im Gehirn mittels sensorischer Stimulation.

Zur Diagnose der Reizverarbeitung des Gehirns werden typischerweise Stimulationsverfahren, wie Dauerreizung, mehrmalige Einzelreizung und periodische Reizung eingesetzt. Als Dauerreizung kommt beispielsweise ein andauernder Ton oder ein visuelles Muster in Frage. Eine Einzelreizung führt zum Beispiel zu sogenannten akustisch oder visuell evozierten Potentialen. Als periodische Reizung kann eine Stimulation mit Flickerlicht zur Diagnose einer photosensiblen Epilpsie beispielhaft genannt werden. Aus den beispielsweise mittels Elektroden gemessenen Reizantworten des Gehirns oder des Sinnesorgans und aus den psychophysischen Befunden (z. B. der Anzahl der erkannten Muster oder gehörten Töne) wird auf die Funktionsweise des untersuchten Sinnessystems geschlossen.

Beim Biofeedback-Training werden optisch oder akustisch dargestellte Feedbackeffekte therapeutisch genutzt, um dem Patienten das Ergebnis willentlich gesteuerter Aktionen auf seine Körperfunktionen, insbesondere sein Vegetativum, rückzumelden und bewußt zu machen. Die Feedback-Signale ermöglichen dabei eine Eigenkontrolle und steigern die Beeinflussung der betroffenen Körperfunktionen durch den Patienten. Angewandt wird das Biofeedback-Training beispielsweise bei funktionellen Herzbeschwerden und neuromuskulären Spannungszuständen. Mit den herkömmlichen diagnostischen Verfahren wird die Abhängigkeit zwischen Reizantwort und vorhandener Aktivität nicht detailliert untersucht. Es werden nur wenige Parameter zerebraler Aktivität erfaßt. Mit den Standardverfahren ist es nicht möglich, die Stimulation auf die vorhandene rhythmische Hirnaktivität beim individuellen Patienten anzupassen, um so einen wesentlich weiteren Funktions- und Antwortbereich abzutasten. Insbesondere ist es nicht möglich, die Auswirkungen gezielter Manipulationen rhythmischer zerebraler Aktivität in unterschiedlichen Frequenzbereichen (zum Beispiel deren Amplitudendämpfung) und Hirnarealen auf die Informationsverarbeitung zu untersuchen.

Das Biofeedback-Training setzt voraus, daß der Patient die zu verbessernde Körperfunktion willentlich geeignet beeinflussen kann und will. Dies ist bei den meisten Organsystemen und bei vielen Hirnfunktionen nicht bzw. nicht in ausreichendem Maß der Fall. Erschwerend kommt hinzu, daß Patienten mit zerebralen Erkrankungen, z.B. Neglect-Patienten nach einem Hirninfarkt, krankheitsbedingt Aufmerksamkeitsstörungen haben können, die eine willentliche Beeinflussung selbst einfacher Körperfunktionen erschweren oder sogar unmöglich machen. So können Neglect-Patienten, die Teile ihres Körpers als nicht zu ihrem Körper gehörig empfinden, kaum zu Übungen mit diesen vernachlässigten Körperpartien bewegt werden.

In der Druckschrift US 4,201,224 ist eine Vorrichtung zur visuellen, auditorischen und taktilen Stimulation beschrieben.

Es ist daher die Aufgabe der Erfindung, eine Vorrichtung zu schaffen, die die physiologische beziehungsweise pathologische neuronale rhythmische Aktivität des Gehirns bedarfsgerecht moduliert. Die Vorrichtung soll Funktionsstörungen des Gehirns diagnostisch sichern und geeignet sein, die Symptomatik zu mildern oder aufzuheben. Die Vorrichtung soll es ermöglichen, Hirnaktivität, die für die sensorische Informationsverarbeitung relevant ist, zu diagnostischen und therapeutischen Zwecken zu untersuchen und zu manipulieren. Weiterhin soll die Vorrichtung so arbeiten, daß bei manchen Patienten, die krankheitsbedingt mindestens eine Körperfunktion nicht mehr willentlich beeinflussen können, die Kontrolle der betroffenen Körperfunktion verbessert bzw. wiederhergestellt wird.

Ausgehend vom Oberbegriff des Anspruchs 1 wird die Aufgabe gelöst durch die im kennzeichnenden Teil des Anspruchs 1 angegebenen Merkmale.

Mit der erfindungsgemäßen Vorrichtung ist es nunmehr möglich, physiologische oder pathologische neuronale rhythmische Aktivität des Gehirns bedarfsgerecht zu modulieren, so daß sie der natürlichen Funktion näherkommt bzw. mit ihr identisch ist. Die Vorrichtung ist geeignet, Funktionsstörungen des Gehirns diagnostisch zu sichern, die Symptomatik zu mildern oder aufzuheben. Die Vorrichtung ermöglicht ein neues diagnostisches Verfahren, bei dem - angepasst auf die vorhandene rhythmische Hirnaktivität beim Patienten - eine gezielte Manipulation rhythmischer Aktivität in verschiedenen Frequenzbereichen durchgeführt wird. Auf diese Weise kann die neuronale Informationsverarbeitung zu diagnostischen und therapeutischen Zwecken untersucht und moduliert werden. Weiterhin arbeitet die erfindungsgemäße Vorrichtung so, daß das Problem, daß manche Patienten manche Körperfunktionen nicht willentlich beeinflussen können, überwunden wird.

Vorteilhafte Weiterbildungen der Erfindung sind in den Unteransprüchen angegeben.

Die Zeichnungen zeigen eine beispielhafte Ausgestaltung der erfindungsgemäßen Vorrichtung als Blockschema bei einem Patienten, sowie einige zur Diagnose und Behandlung einzusetzende Pulsfolgen.

Es zeigt:

Fig:1:  Ein Blockschema der Vorrichtung.

Fig.2:  Stimulusabfolge zur Anregung mit der Resonanzfrequenz, auf die zum Zwecke der Desynchronisation ein in der vulnerablen Phase applizierter Einzelpuls folgt.

Fig.3a:  Beispielhafter Verlauf für die mit dem Mittel zur Erzeugung der senso- rischen Reize 1 erzeugten zeitli- chen Muster des sensorischen Rei- zes.

Fig.3b: Die der Darstellung in Figur 3a zu- gehörige schematische Abbildung des Aktivitätsmusters der erkrankten Hirnregion.

Fig.4a: Scan der Anregungsfrequenz, bei dem die Frequenz der Pulsfolge langsam variiert wird.

Fig.4b: Ansteigen der natürlichen rhythmi- schen Aktivität.

Fig.5a-f: Schematische Abbildung der zu einem Eichprozeß gehörenden Phase resetting-Kurven.

Fig.6: Flußdiagramm für die erfindungsge- mäße Arbeitsweise der Vorrichtung.

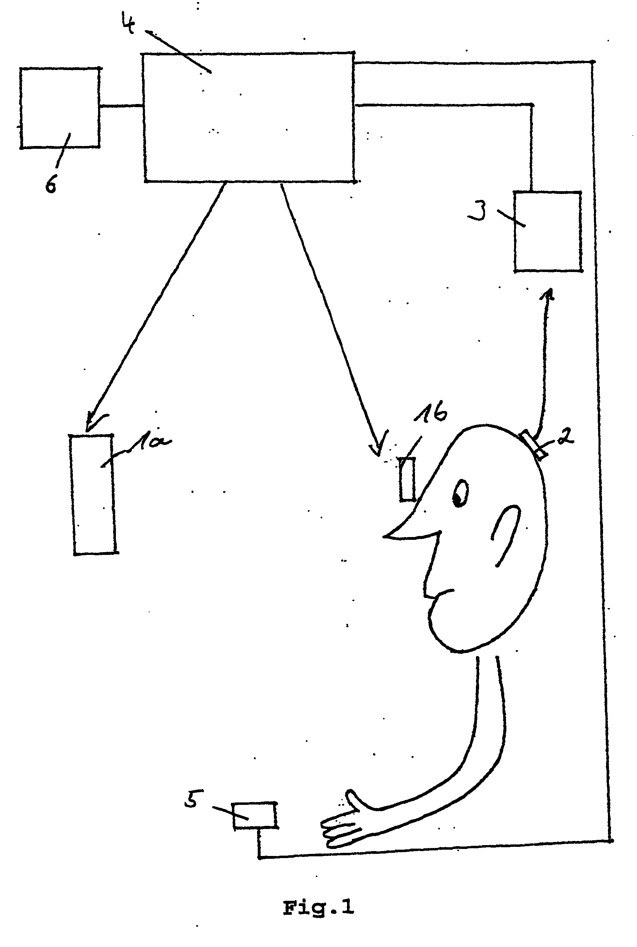

Figur 1 zeigt eine Vorrichtung, mit einem Stimulator 1 (1a, 1b), vor dem ein Patient sitzt. Am Kopf des Patienten ist ein Sensor 2 angebracht, der über einen Trennverstärker 3 an eine Steuereinheit 4 angeschlossen ist. Die Vorrichtung umfaßt einen Empfänger 5, der ebenfalls an die Steuereinheit 4 angeschlossen ist und der Reaktionen des Patienten registrieren kann. Außerdem umfaßt die Vorrichtung ein Mittel zur Überprüfung der Stimulation 6, das über Mittel zur Datenverarbeitung und zur Darstellung der Daten verfügt, so daß die Ergebnisse für den Untersucher visuell und/oder auditorisch dargestellt werden können. Die Steuereinheit 4 steht mit einem Mittel zur Überprüfung der Stimulation 6 in Verbindung. Der Sensor 2, der Empfänger 5, der Stimulator 1 sowie das Mittel zur Überprüfung der Stimulation 6 können auch kontaktfrei, zum Beispiel über Sender und Empfänger, mit der Steuereinheit 4 in Verbindung stehen.

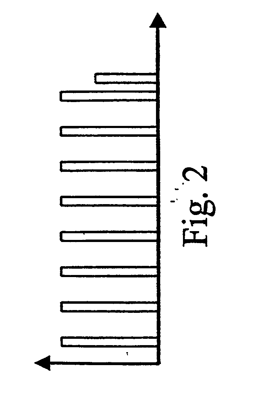

Figur 2 zeigt ein schematisches Muster einer Pulsfolge für eine repetitive Anwendung. Diese Pulsfolge besteht aus einer periodischen Pulsfolge und einem Desynchronisationspuls (letzter Puls). Die Frequenz der periodischen Pulsfolge ist die Resonanzfrequenz des zu desynchronisierenden Rhythmus. Zweck der periodischen Pulsfolge ist es, ein Entrainment durchzuführen, welches bewirkt, daß die Phasendynamik des zu desynchronisiernden Rhythmus kontrolliert wird. Hierdurch trifft der nach einer konstanten Zeitverzögerung applizierte Desynchronisationspuls den neuronalen Rhythmus verläßlich in seine vulnerablen Phase. Die Abszisse ist eine Zeitachse in willkürlichen Einheiten und die Ordinate gibt die Intensität der Reize in willkürlichen Einheiten wieder.

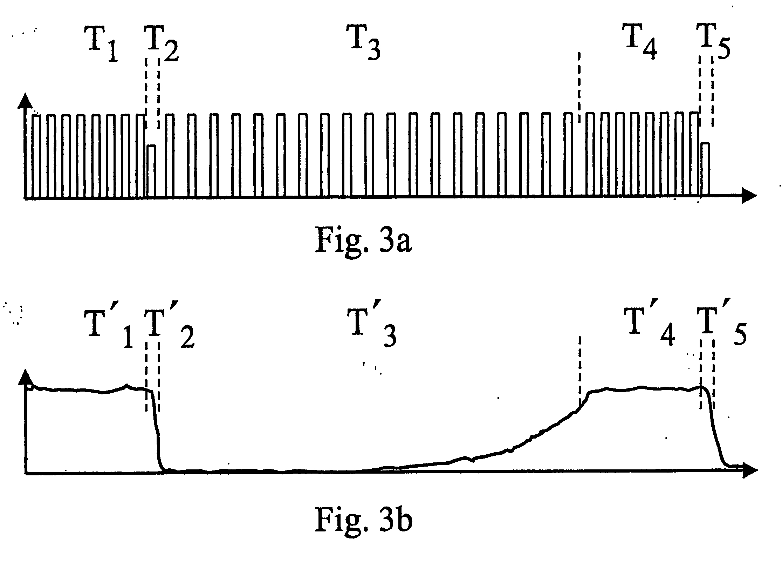

In Figur 3a ist die Abszisse eine Zeitachse in willkürlichen Einheiten und die Ordinate gibt die Intensität der Reize in willkürlichen Einheiten wieder. Die Zeitabschnitte $T_1$ und $T_2$ sowie $T_4$ und $T_5$ entsprechen der Abbildung der Figur 2. In Zeitabschnitt $T_3$ wird eine periodische Reizfolge appliziert, deren Frequenz hinreichend von der Resonanzfrequenz der zu desynchronisierenden Neuronenpopulation verschieden ist. In den Zeitabschnitten $T_1$ und $T_2$ sowie $T_4$ und $T_5$ wird jeweils die in Figur 2 dargestellte desynchronisierende Stimulation durchgeführt.

In Figur 3b ist die Abszisse die Zeitachse mit den selben Zeiteinheiten wie in Figur 3a. Die Ordinate zeigt schematisch die in einem gleitenden Zeitfenster zeitlich gemittelte Amplitude des zu desynchronisierenden Rhythmus in willkürlichen Einheiten. Die Zeitabschnitte $T_k$' sind identisch mit den Zeitabschnitten $T_k$, wobei k=1,2,3,4,5 ist. Während des Entrainments in Zeitabschnitt $T_1$ kommt es neben der Kontrolle der Phasendynamik zusätzlich zu einer resonanzartigen Verstärkung der Amplitude. Der desynchronisierende Einzelreiz im Zeitabschnitt $T_2$' trifft den neuronalen Rhyth- mus in seiner vulnerablen Phase und desynchronisiert ihn, wodurch am Ende dieser Stimulation die Amplitude minimiert ist. Im Zeitabschnitt $T_3$ wird weiter sensorisch Stimuliert, so daß der Patient die von ihm auszuführende Aufgabe, zum Beispiel das Auffinden spezieller Muster, fortlaufend durchführen kann. Um das Aufschaukeln des pathologischen Rhythmus möglichst lange herauszuzögern wird in Zeitabschnitt $T_3$' mit einer von der Resonanzfrequenz verschiedenen Frequenz periodisch gereizt. Sobald die Amplitude des zu desynchronisierenden Rhythmus wieder einen Schwellenwert überschreitet, wird erneut eine Desynchronisation durchgeführt, wobei die Stimulation in den Zeitabschnitten $T_4$' und $T_5$' identisch ist mit der Stimulation in den Zeitabschnitten $T_1$' und $T_2$'.

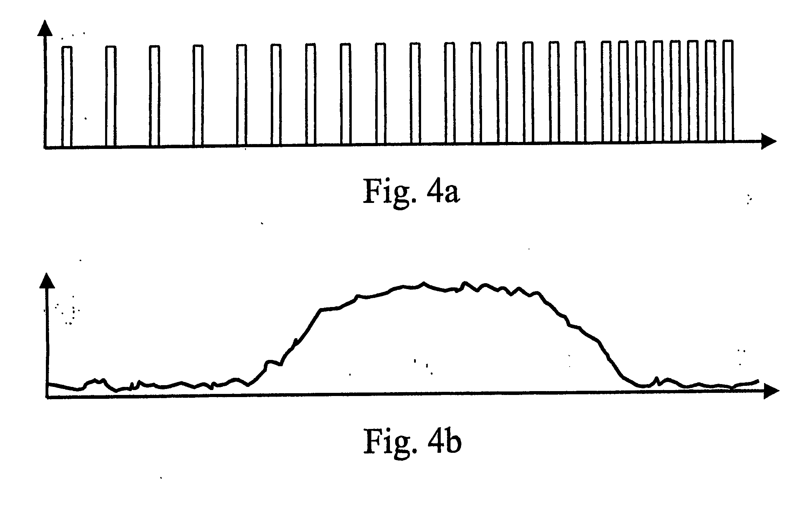

In Figur 4a ist die Abszisse eine Zeitachse in willkürlichen Einheiten und die Ordinate gibt die Intensität der Reize in willkürlichen Einheiten wieder. Figur 4a zeigt schematisch die für den Frequenzscan verwendete Stimulation. Dabei wird eine periodische Reizfolge appliziert, deren Frequenz langsam variiert wird, hier zum Beispiel langsam anwächst.

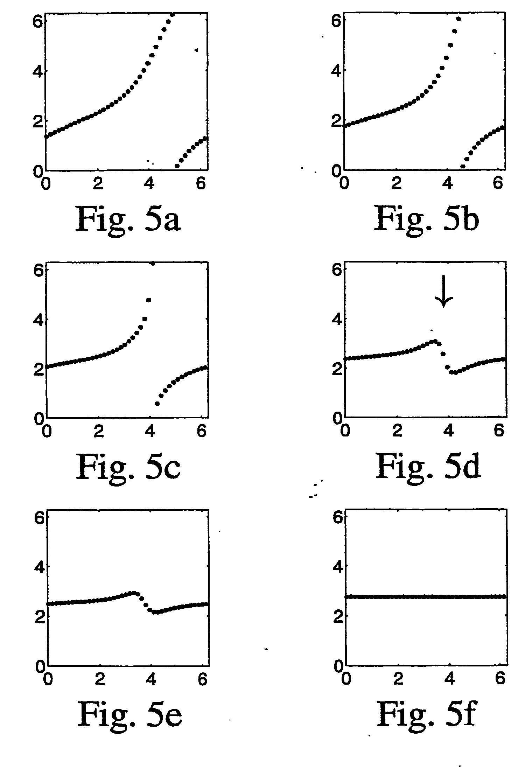

In Figur 4b ist die Abszisse die Zeitachse mit den selben Zeiteinheiten wie in Figur 4a. Die Ordinate zeigt schematisch die in einem gleitenden Zeitfenster zeitlich gemittelte Amplitude des zu desynchronisierenden Rhythmus in willkürlichen Einheiten. Entspricht die Anregungsfrequenz der in Figur 4a dargestellten Reizfolge einer Resonanzfrequenz, so kommt es zur Resonanz, das heißt, die Amplitude des neuronalen Rhythmus wächst an. Figur 5 zeigt Phasen- resetting-Kurven, bei denen $\varphi_e$ über $\varphi_b$ dargestellt wird. $\varphi_e$ ist dabei die Phase der neuronalen Aktivität, die entweder unmittelbar nach Stimulation oder mit einer konstanten Zeitverzögerung nach Stimulation bestimmt wird. $\varphi_b$ ist die Phase der neuronalen Aktivität, die entweder unmittelbar zum Zeitpunkt des Stimulationsbeginns oder mit einer konstanten Zeitverzögerung vor Stimulationsbeginn bestimmt wird. Die Phasen $\varphi_e$ und $\varphi_b$ werden im Gradmaß modulo $2\pi$ ange- geben. Jede Teilabbildung a)-f) entspricht einer Serie von Testreizen, bei denen derselbe Stimulus, das heißt ein Reiz mit konstanter Intensität und Reizdauer, bei unterschiedlichen Werten der Anfangsphase $\varphi_b$ appliziert werden. Die Wirkung des Reizes auf die Phasendynamik des zu desynchronisierenden neuronalen Rhythmus wird mittels der Phasen- resetting-Kurven ausgewertet. In Teilabbildungen a) bis c) ist der mittlere Gradient der Kurve gleich 1, während in Teilabbildungen d) bis f) ist der mittlere Gradient der Phasen-resetting-Kurve gleich null. Unter einem mittleren Gradient ist der über eine Periode von $\varphi_b$ gemittelte Gradient gemeint. Der Übergang zwischen einer Phasen-resetting-Kurve mit mittlerem Gradienten gleich 1 zu einer Phasen-resetting-Kurve mit mittlerem Gradienten gleich 0 findet zwischen Teilabbildungen c) und d) bei der durch den vertikalen Pfeil hervorgehobenen Phase $\varphi_b$ statt. Dieser Wert der Phase $\varphi_b$ ist die vulnerable Phase des zu desynchronisierenden neuronalen Rhythmus. Der optimale Wert für die Intensität liegt zwischen den beiden Intensitätswerten der Teilabbildungen c) und d). Um diesen Wert zu erhalten kann man entweder näherungsweise den Mittelwert der Intensitäten von c) und d) wählen oder genauer noch weitere Phasen-resetting-Kurven mit Intensitätswerten zwischen denen von c) und d) anfertigen.

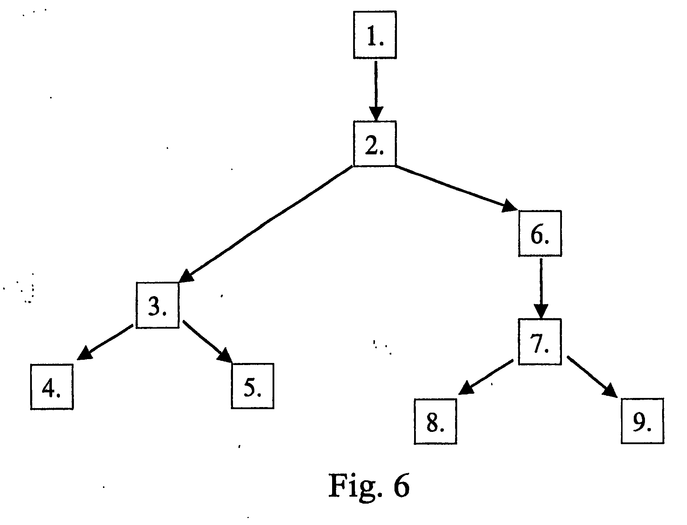

Fig. 6: Flußdiagramm für die erfindungsgemäße Verfahrensweise:

Figur 6 zeigt ein Flußdiagramm der erfindungsgemäßen Verfahrensweise.

Zuerst erfolgt eine Bestimmung des Frequenzspektrums unter Spontanbedingungen (1) (d.h. ohne Stimulation), wobei der Patient entspannt ist und beispielsweise während 5 min die Augen offen und während weiterer 5 min die Augen geschlossen hat. Bei offenen bzw. geschlossenen Augen sind jeweils bestimmte Hirnrhythmen besonders stark bzw. besonders schwach ausgeprägt. Z.B. ist der alpha-Rhythmus bei geschlossenen Augen typischerweise stärker ausgeprägt, bei offenen Augen hingegen schwächer ausgeprägt. Eine starke Ausprägung eines neuronalen Rhythmus bedeutet, daß dieser Rhythmus insbesondere eine große Amplitude hat. Auf diese Weise wird die ohne Stimulation auftretende Bandbreite der Ausprägung der physiologischen bzw. pathologischen Rhythmen ermittelt.

Als nächstes erfolgt die Durchführung eines Frequenzscans (Auswertung der Stärke der Resonanz mittels Amplitudenbestimmung des angeregten Rhythmus), evtl. zusätzlich Bestimmung der Güte des Entrainments über Bestimmung der Stärke der Phasensynchronisation zwischen der Reizfolge und dem angeregten Rhythmus.

In Abhängigkeit von den Ergebnissen von (1) und (2) erfolgt nun jeweils ein unterschiedliches weiteres Vorgehen. Falls bei dem Patienten natürliche, nicht krankhafte rhythmische Aktivitäten zu schwach ausgeprägt oder überhaupt nicht vorhanden sind, erfolgt eine bedarfsgesteuerte Synchronisation (3-5). Falls sich bei dem Patienten eine pathologische rhythmische Aktivität findet, erfolgt eine bedarfsgesteuerte Desynchronisation (6-9).

Die bedarfsgesteuerte Synchronisation (3) kann auf zweierlei Weise erfolgen: Im Rahmen einer einfachen Kontrollfunktion wird zu Beginn der sensorischen Stimulation die Anregungsfrequenz $f_A$ und die Intensität festgelegt und während der Stimulation konstant gehalten (4). In einer vorzugsweisen Ausführung der Erfindung wird die Stimulation mit gemäß (2) geeigneten Werten der Anregungsfrequenz $f_A$ und der Intensität begonnen (5). Steuereinheit 4 paßt aber in diesem Modus (5) die Parameter (insbesondere die Intensität) bedarfsgesteuert an.

Für die bedarfsgesteuerte Desynchronisation wird zuerst die Güte des Entrainments überprüft (6). Dann erfolgt die Bestimmung der vulnerablen Phase (7), welche - wie unten beschrieben - mit einer Bestimmung der optimalen Reizintensität bzw. Reizdauer verbunden ist. Die bedarfsgesteuerte Desynchronisation kann nun auf zwei Weisen erfolgen: Entweder wird eine repetitive Anwendung der sensorischen Stimuli (8) durchgeführt oder es wird eine anhaltende Anwendung durchgeführt (9). Während der repetitiven Anwendung (8) wird dieselbe desynchronisierende Reizfolge repetitiv angeboten, wobei in den Pausen dazwischen keine Reizdarbietung erfolgt. Während der andauernden Anwendung (9) hingegen wird dauerhaft sensorisch stimuliert, wobei beim Überschreiten des Schwellenwertes der zu desynchronisierenden neuronalen Aktivität immer dieselbe desynchronisierende Reizfolge appliziert wird.

Bei praktisch allen Teilschritten kann und soll vorzugsweise über das Mittel zur Visualisierung (Fig.1 Bez.6) ein Feedback an den Untersucher gegeben werden können.

Im Folgenden sollen die einzelnen Bestandteile der erfindungsgemäßen Vorrichtung sowie deren Funktionsweise erläutert werden.

Bei dem Stimulator 1 handelt es sich um Reizgeber, der Signale erzeugt, die vom Patienten bewusst oder unbewusst wahrgenommen werden können. Grundsätzlich können dabei alle vom Patienten sensorisch verarbeitbaren Signale erzeugt werden. Beispielhaft können visuelle, akustische Reize oder Reize, die den Tastsinn oder - weniger wahrscheinlich - die Schmerzempfindung anregen, genannt werden. Visuelle Reize können Bilder oder Muster sein. Die visuellen Reize können beispielsweise über einen speziellen Bildschirm 1a oder eine Shutterbrille 1b abgegeben werden. Bei dem Bildschirm handelt es sich vorzugsweise um einen Projektionsschirm, der über einen Shutterverschluß mit einem Projektor, der ein zeitlich kontinuierliches Bild erzeugt, verbunden ist. Der Verschlußmechanismus der Shutterbrille und des Shutterverschlusses für den Projektor arbeiten vorzugsweise entweder nach dem LCD-Verfahren oder dem FLC (ferroelectric liquid cristal)-Verfahren. Die zur Ausübung visueller Reize eingesetzten Bilder und Muster sind dem Fachmann bekannt. Dies sind beispielsweise Kanisza-Figuren.

Als akustische Reize können alle Töne oder komplexe Geräusche, wie Iteration von zeitverzögerten breitbandigen Rauschen oder im hörbaren Frequenzbereich dienen, die über einen Lautsprecher 1c oder einen Kopfhörer 1d abgegeben werden. Ein Reizgeber, der den Tastsinn oder das Schmerzempfinden anregt kann beispielsweise ein somatosensorischer Stimulationsgenerator 1e oder ein zeitlich modulierter Laser 1f sein. Ein Reizgeber im Sinne der Erfindung ist daher ein Mittel zur Erzeugung eines visuellen, akustischen, oder eines anderen sensorischen Signals, bzw. Stimulus. Der Stimulator 1 kann die genannten Signale in einem zeitlichen Muster entweder rhythmisch oder arhythmisch abgeben. Das bedeutet, daß visuelle Bilder oder Muster in periodischen zeitlichen Abfolgen von vorzugsweise 1 bis 100 Hz oder 1 bis 70 Hz und/oder in komplexen, nicht periodischen zeitlichen Abfolgen erzeugt werden können, jedoch sind die Anwendungen nicht auf diese Frequenzen beschränkt. Weiterhin kann auch die Intensität bzw.

Amplitude der Signale variieren. Bei visuellen Reizen kann neben der Helligkeit auch der Kontrast variiert werden. Analog können Töne in periodischen zeitlichen Abfolgen von vorzugsweise 1 bis 100 Hz und/oder in komplexen, nicht periodischen zeitlichen Abfolgen appliziert werden. Hierbei kann die Lautstärke variiert werden. Analoges gilt für die Mittel zur Erzeugung der anderen sensorischen Reize, bei denen Druck und Frequenz variiert werden können. Die komplexen, nicht periodischen zeitlichen Abfolgen von sensorischen Einzelreizen können - wie unten beschrieben - zum Beispiel aus einer Kombination einer periodischen Reizabfolge mit einem nachfolgenden, qualitativ verschiedenen Einzelreiz sein.

Beim Gesunden findet sich typischerweise in bestimmten Frequenzbändern rhythmische Aktivität, die jeweils bevorzugt in bestimmten Hirnarealen vorkommt. Zum Beispiel beobachtet man den sogenannten $\alpha$-Rhythmus (ca. 10Hz) bevorzugt im Bereich der visuellen Hirnrindenareale. Bei Patienten können einerseits diese physiologischen Rhythmen vermindert ausgeprägt sein oder andererseits pathologische Rhythmen vorhanden sein, die in einem untypischen, das heißt unphysiologischen Frequenzband auftreten. Ein pathologischer Rhythmus kann auch durch normalen Frequenzgehalt aber untypische anatomische Lokalisation gekennzeichnet sein. Ein pathologischer Rhythmus muß nicht nur auf eine einzige Hirnregion beschränkt sein, sondern kann auch andere, anatomisch verbundene Hirnregionen durch Einspeisen der krankhaften rhythmischen Aktivität in ihrer Funktionsweise empfindlich stören.

Nachdem der Frequenzgehalt der Hirnaktivität des Patienten durch den Untersucher charakterisiert ist, werden entweder zu schwach ausgeprägte physiologische Rhythmen angeregt oder zu stark ausgeprägte pathologische Rhythmen unterdrückt bzw. abgeschwächt. Sind pathologische Rhythmen schwach ausgeprägt, kann durch vorwiegend periodische Stimuli, welche vom Stimulator 1 abgegeben werden, diese Rhythmen angeregt werden, was der Diagnose dient. In einem weiteren Schritt kann durch Stimuli eine Desynchronisation der krankhaften rhythmischen Aktivitäten bewirkt werden. Dabei sind die Signalabfolgen, die die Desynchronisation bewirken, verschieden von denen, die die Analyse bzw. Diagnose ermöglichen, indem sie die krankhaften rhythmischen Aktivitäten verstärken. Zur Desynchronisation wird mindestens ein desynchronisierender Puls erzeugt.

Die mittels des Stimulators 1 abgegebenen Signale modulieren rhythmische Aktivität in bestimmten Hirnarealen, die über den Sensor 2 detektiert werden können. Sensor 2 ist in diesem Sinne ein Mittel zur Erfassung von Hirnaktivitäten. Als Beispiele können Skalp-EEG-Elektroden oder MEG-Sensoren, d.h. SQUIDS, genannt werden. Die Vorrichtung ist erfindungsgemäß mit mindestens einem Sensor 2 ausgestattet, der mit der Steuereinheit 4 in Verbindung steht.

Steuereinheit 4 verarbeitet die von dem Sensor 2 weitergegebenen Signale. Die Steuereinheit 4 verfügt über Mittel zur Durchführung der in der Anmeldung beschriebenen Verfahrensschritte. Diese Mittel sind insbesondere ein Computer oder eine elektronische Schaltung sowie ein Computerprogramm oder ein programmierbarer Prozessor, wie zum Beispiel ein FPGA (Field programmable array), welche dazu in der Lage sind, die erfindungsgemäßen Schritte der Signalerfassung und der Auswertung durchzuführen und den Stimulator 1 in der erfindungsgemäßen Weise anzusteuern. Es ist besonders zweckmäßig, das Verfahren auf geeigneten Prozessoren durchzuführen. Der Begriff Prozessor ist dabei in keiner Weise einschränkend zu verstehen. Es kann sich hierbei um eine beliebige zur Durchführung von Berechnungen geeignete Einheit handeln. Es ist möglich, daß der Prozessor aus mehreren Einzelprozessoren besteht, die vorzugsweise zu einer geeigneten Prozessoreinheit zusammengefaßt sind.

Im Sinne der vorliegenden Erfindung kann ferner jede zur Durchführung von Berechnungen geeignete Schaltung eingesetzt werden. Zweckmäßigerweise ist die Schaltung in einem Computer oder in einem Logikbaustein eingebaut. Die in der Beschreibung angegebenen Mittel zur Durchführung der erfindungsgemäßen Verfahrensschritte sind Bestandteile der Steuereinheit 4, umfassend mindestens eine Komponente aus der Gruppe bestehend aus einem Computer, einer elektronischen Schaltung, einem Computerprogramm bzw. einem Prozessor. Die Mittel zur Steuerung der unterschiedlichen Verfahrensschritte müssen nicht zwingend in einem einzigen Gerät untergebracht sein.

Steuereinheit 4 ermittelt den Ausprägungsgrad einer pathologischen rhythmischen Aktivität. Ist die krankhafte Aktivität nicht oder nur minimal vorhanden, so gibt die Steuereinheit 4 Steuersignale an den Stimulator, welcher dann entweder keine oder andere Stimuli aussendet, die sich entweder in der Frequenz, der Amplitude oder und Frequenz und Amplitude von den vorhergehenden Stimuli unterscheiden. Im diagnostischen Anwendungsbereich werden Frequenz und/oder Amplitude der Stimuli so lange verändert, bis die pathologische Reaktion maximal ist, das heißt, die rhythmische Reaktion des kranken Hirnareals am stärksten ist. Dies hat den Vorteil, daß ansonsten möglicherweise nicht erkennbaren pathologischen Rhythmen unter Umständen erkannt werden können, falls sie zum Zeitpunkt der diagnostischen Untersuchung gerade zu schwach ausgeprägt sind. Hierzu verfügt die Steuereinheit 4 über Mittel zum Hervorrufen einer maximalen physiologischen und/oder pathologischen Hirnaktivität. Dieses Mittel verfügt beispielsweise über eine elektronische Schaltung, einen Prozessor oder einen Computer und zugehörige Software, welche sicherstellen, daß Stimulationsfolgen wie im Folgenden beschrieben durchgeführt werden. Die krankhaften rhythmischen Aktivitätsmuster werden von der Steuereinheit 4 analysiert. Daraufhin ermittelt die Steuereinheit 4 ein anderes zeitliches Muster des Stimulus, das geeignet ist, die krankhafte Aktivität gezielt zu modulieren und insbesondere das krankhafte Aktivitätsmuster zu unterdrücken oder abzuschwächen. Damit wird bewirkt, daß in Umkehrung des ersten Effektes, nämlich der Förderung der pathologischen Aktivität, eine Dämpfung und - besonders bevorzugt - eine vollständige Unterdrückung der krankhaften Hirnaktivität erfolgt. Wiederum detektiert der Sensor 2 die Hirnaktivität und die Steuereinheit 4 analysiert das neue Verhalten des Gehirns. Durch mehrere Zyklen dieser Art ermittelt die Steuereinheit 4 im therapeutischen Teil der Anwendung, mit welchen Stimuli das krankhafte Verhalten möglichst vollkommen unterdrückt werden kann. Mit der Steuereinheit 4 steht weiterhin der Empfänger 5 in Verbindung, der zur Kontrolle des Patienten dient. Beim Empfänger 5 im Sinne der Erfindung handelt es sich beispielsweise um einen Taster oder einen Schalter oder Hebel, der vom Patienten bedient wird. Der Patient erhält die Anweisung, den Empfänger 5 auf bestimmte Signale hin zu betätigen. Damit kann die Aufmerksamkeit des Patienten, seine Fähigkeit zur Verarbeitung sensorischer Reize bzw. der Erfolg der Behandlung kontrolliert werden. Die Signale von Empfänger 5 werden in der Steuereinheit 4 verrechnet und an das Mittel zur Überprüfung der Stimulation 6 weitergeleitet. Über dieses Mittel 6 kann der Untersucher die Güte der Stimulation und die Durchführung der gestellten Aufgabe durch den Patienten überprüfen. Die mit dem Empfänger 5 und dem Mittel zur Überprüfung der Stimulation 6 ausgestattete erfindungsgemäße Vorrichtung stellt somit eine bevorzugte Ausführungsform der Erfindung dar.

Im Falle der Anwendung kann zwischen zwei Fallgestaltungen A und B unterschieden werden die im Folgenden beispielhaft erläutert werden.

A: Bei Patienten sind natürliche, nicht krankhafte, rhythmische Aktivitäten zu schwach ausgeprägt oder überhaupt nicht vorhanden.
B: Der Patient zeigt eine pathologische rhythmische Aktivität mindestens einer Hirnregion.

Für die Fallgestaltungen A und B arbeitet die Steuereinheit 4 in folgender Weise:

Frequenzscan:

Der Frequenzscan wird sowohl bei Fallgestaltung A als auch bei Fallgestaltung B als Erstes durchgeführt. Beim Frequenzscan wird eine periodische sensorische Stimulation mit einer Anregungsfrequenz $f_A$ vorgenommen, wobei $f_A$ langsam zwischen vorzugsweise 1 und 100 Hz, besonders bevorzugt zwischen 1 bis 60 Hz variiert wird. In Figur 4a ist dies beispielhaft durch die mit zunehmender Frequenz applizierte Signalfolge wiedergegeben. Sensor 2 mißt die neuronale Aktivität und leitet sie an Steuereinheit 4 weiter, die ermittelt in welchem Frequenzbereich der neuronalen Aktivität es zu einer Anregung kommt. Die Anregung kann dabei quantifiziert werden durch

(i) durch die über den angeregten Frequenzbereich integrierte Amplitude des Powerspektrums oder analog dazu
(ii) über die mittels der Hilbert-Transformation bestimmte instantane Amplitude des Frequenzbereichs.

Die erfindungsgemäße Vorrichtung umfaßt daher Mittel zur Durchführung eines Frequenzscans sowie zur Durchführung der Schritte (i) und/oder (ii).

Eine hier beispielhaft angeführte elektronische Schaltung beziehungsweise äquivalente Mittel in Steuereinheit 4 sowie das Computerprogramm, welche beispielhaft nach den Methoden (i) und (ii) arbeiten, können als Mittel zur Quantifizierung der neuronalen Aktivität dienen.

Dieser Frequenzscan wird durch die Steuerung 4 durchgeführt, die das Mittel zur Erzeugung von sensorischen Reizen 1 dazu aktiviert, die jeweilige Frequenz in Form eines sensorischen Reizes an den Patienten weiterzugeben. Hierzu verfügt die Steuereinheit 4 über Mittel zum Ansteuern des Stimulators 2, beispielsweise einen TTL-Pulsgeber. Die Steuereinheit 4 erkennt dann durch die von dem Sensor 2 erfaßten Signalen bzw. deren Amplitude in den untersuchten Frequenzbereichen bei welchen Anregungsfrequenzen eine maximale Anregung erfolgt.Die Vorrichtung umfaßt vorzugsweise daher Mittel, die dazu in der Lage sind, in den mit Sensor 2 gemessenen Signalen neben dem Frequenzbereich der Anregungsfrequenz noch andere Frequenzbereiche zu untersuchen. Diese Mittel können eine zeitabhängige Frequenzanalyse auf der Basis der Fourier-Transformation oder der Wavelet-Analyse sein. Hierzu umfaßt die Steuereinheit 4 ein Mittel, das zur Durchführung dieser Schritte geeignet ist. Hierbei handelt es sich, wie weiter oben beispielhaft angegeben, um einen Computer, eine elektronische Schaltung, einen Prozessor, eine programmierbare elektronische Schaltung (FPGA) oder ein Computerprogramm. Die Frequenz der angeregten Aktivität kann dabei mit der Anregungsfrequenz übereinstimmen oder auch nicht. Überraschenderweise hat sich heraus gestellt, daß die Frequenz der entrainenden periodischen Reizfolge folgender Gesetzmäßigkeit gehorcht:

$$\frac{f_R}{f_A} = \frac{n}{m} \qquad\qquad \text{Formel 1}$$

mit

$f_A$ = Anregungsfrequenz, das heißt die Frequenz der anregenden periodischen Reizfolge

$f_R$ = Frequenz der angeregten neuronalen Ak- tivität (Resonanzfrequenz)

wobei n und m ganzzahlig und klein, das heißt $\leq 10$, (nämlich 1,2,3,4,5,6,7,8,9,10) sind, z.B. n/m = 1/1, ½, 2/3 etc..

Mit Hilfe des Frequenzscans werden zwei Aspekte des Anregungsverhaltens untersucht:

1.) Es wird untersucht, ob eine Anregung physiologischer Rhythmen in den für diese Rhythmen zu erwartenden Frequenzbereichen auftritt. Bei Flickerlichtstimulationen wären dies beispielsweise Frequenzen im Bereich von 10 Hz, 20 Hz, 40 Hz und 80 Hz. Hierdurch wird ermittelt, ob ein physiologischer Rhythmus, der krankheitsbedingt unter Spontanbedingungen, d.h. ohne Stimulation, zu schwach ausgeprägt ist, durch periodische Stimulation angeregt werden kann.

2.) Es wird untersucht, ob eine Anregung eines pathologischen Rhythmus auftritt. Letzterer kennzeichnet sich vornehmlich dadurch, daß er in einem unphysiologischen Frequenzbereich auftritt oder, daß er zwar in einem physiologischen Frequenzbereich, dafür aber in einem untypischen Hirnareal auftritt. Die physiologischen Frequenzbereiche sind die Frequenzbereiche, in denen neuronale Rhythmen natürlicherweise vorkommen. Beispielhaft können der $\alpha$-Rhythmus im Bereich um 10 Hz und der $\beta$-Rhythmus im Bereich um 20 Hz genannt werden. Auf diese Weise wird ermittelt, ob ein krankheitsbedingter Rhythmus durch periodische Stimulation angeregt werden kann. Ein solcher pathologischer Rhythmus ist dabei typischerweise, aber nicht zwingend schon unter Spontanbedingungen, das heißt ohne Stimulation, vorhanden.

Nachdem der Frequenzscan wie oben angegeben durchgeführt worden ist, erfolgt die Anwendung gemäß den Fallgestaltungen A und B.

A Bedarfsgesteuerte Synchronisation:

Ziel der bedarfsgesteuerten Synchronisation ist es, bei Patienten, die einen oder mehrere zu schwach ausgeprägte physiologische Rhythmen aufweisen, diese während der Verarbeitung sensorischer Reize anzuregen. Hierdurch soll die Reizverarbeitung, die durch den geschwächten physiologischen Rhythmus gestört ist, verbessert bzw. ermöglicht werden. Hierzu registriert Sensor 2 die neuronale Aktivität des anzuregenden Hirnareals. Das von Sensor 2 gemessene Signal wird vorzugsweise über den Trennverstärker 3 an die Steuereinheit 4 weitergeleitet. Die Steuereinheit 4 kann dabei auf zwei verschiedene Weisen die sensorische Stimulation kontrollieren:

1.) Im Rahmen einer einfachen Kontrollfunktion wird zu Beginn der sensorischen Reizung die Anregungsfrequenz $f_A$ und die Intensität der anregenden sensorischen Reize gemäß der Ergebnisse des Frequenzscans festgelegt. Diese Stimulationsparameter bleiben dann während der sensorischen Reizung konstant.

2.) Wie unter 1.) wird gemäß der Ergebnisse des Frequenzscans mit geeigneten Werten der Anregungsfrequenz $f_A$ und der Intensität begonnen. Steuereinheit 4 passt diese Parameter während der sensorischen Reizung bedarfsgesteuert an. Das heißt, Steuereinheit 4 reagiert auf eine Abnahme der Amplitude des anzuregenden Rhythmus durch eine Steigerung der Intensität der anregenden Reize. Hierzu verfügt die Steuereinheit 4 über Mittel zum Registrieren der Veränderung der Amplitude des anzuregenden Rhythmus und zur Veränderung der Anregungsintensität. Hierbei handelt es sich, wie weiter oben beispielhaft angegeben, um einen Computer, eine elektronische Schaltung, einen Prozessor, eine programmierbare elektronische Schaltung (FPGA) oder ein Computerprogramm. Der Bereich der hierzu verwendeten Intensität ist aus Sicherheitsgründen, das heißt zur Vermeidung von epileptischen Anfällen, nach oben beschränkt.

Während der wie unter 1.) oder 2.) beschriebenen sensorischen Stimulation werden dem Patienten definierte Reize, wie zum Beispiel Kanisza-Figuren, dargeboten. Der Patient wird zuvor instruiert, nach speziellen Merkmalen in diesen Reizen zu suchen. Über die über den Taster 5 erfolgende Rückmeldung durch den Patienten wird dabei vorzugsweise kontrolliert, ob die Erkennung der dargebotenen sensorischen Reize durch die Anregung des physiologischen Rhythmus verbessert bzw. ermöglicht wird. Bei wenigstens einmaligem, zum Beispiel dreimaligem, Ausbleiben der Reaktion des Patienten wird von der Steuereinheit 4 ein geeignetes Signal an das Mittel zur Überprüfung der Stimulation 6 und damit an den Untersucher weitergeleitet. Dieses Signal dient dazu, dem Untersucher mitzuteilen, daß der Patient nicht willens oder nicht in der Lage ist, die sensorischen Reize gemäß der vorgegebenen Aufgabe zu verarbeiten.

B Bedarfsgesteuerte Desynchronisation:

Ziel der bedarfsgesteuerten Desynchronisation ist es, bei Patienten, die einen oder mehrere zu stark ausgeprägte, pathologische Rhythmen aufweisen, diese während der Verarbeitung sensorischer Reize zu dämpfen bzw. zu unterdrücken. Hierdurch soll die Reizverarbeitung, die durch den zu stark ausgeprägten neuronalen Rhythmus gestört ist, verbessert bzw. ermöglicht werden. Diese Aufgabe wird mit der erfindungsgemäßen Vorrichtung und insbesondere mit Steuereinheit 4, beziehungsweise deren weiter oben beschriebenen ausführenden Mitteln und deren Funktionsweise wie folgt dargestellt gelöst. Sensor 2 registriert zu diesem Zweck die neuronale Aktivität des zu dämpfenden Hirnareals. Das von Sensor 2 gemessene Signal wird vorzugsweise über den Trennverstärker 3 an die Steuereinheit 4 weitergeleitet. Die Steuereinheit 4 arbeitet erfindungsgemäß nach folgendem Prinzip:

Eine rhythmisch aktive Neuronenpopulation kann durch einen sensorischen Reiz desynchronisiert werden, wenn der Reiz einerseits die richtige Intensität und Dauer hat und andererseits in einer kritischen Phase der kollektiven Oszillation der Neuronenpopulation, der sogenannten vulnerablen Phase, appliziert wird. In Folge der unvermeidbaren Variabilität der Frequenz einer Neuronenpopulation, ist es schwierig, die vulnerable Phase sicher zu treffen. Das Problem wird erfindungsgemäß dadurch gelöst, daß komplexere Stimuli verwendet werden.

Diese bestehen aus zwei qualitativ unterschiedlichen Reizen:

Der erste Reiz kontrolliert die Dynamik der Neuronenpopulation so, daß am Ende dieses Reizes der dynamische Zustand der Neuronenpopulation mit hinreichender Genauigkeit bekannt ist. Hierzu wird ein Entrainment durchgeführt, das heißt, eine entrainende, periodische Reizfolge wird appliziert, um die Dynamik der Neuronenpopulation mit der Reizfolge in Takt zu bringen. Hierzu verfügt die erfindungsgemäße Vorrichtung über Mittel zur Durchführung eines Entrainments, das heißt, einer periodischen Stimulation zu dem Zweck, die dem Zweck dient, den Rhythmus, das heißt die Phasendynamik, der angeregten neuronalen Aktivität zu kontrollieren. Hierbei handelt es sich, wie weiter oben beispielhaft angegeben, um einen Computer, eine elektronische Schaltung, einen Prozessor, eine programmierbare elektronische Schaltung (FPGA) oder ein Computerprogramm.

Der zweite Reiz folgt auf den ersten, entrainenden Reiz (=Reizfolge) mit einer im wesentlichen konstanten Zeitverzögerung. Er trifft die pathologisch synchronisierte, rhythmische Neuronenpopulation in einem vulnerablen Zustand und führt auf diese Weise zu einer Desynchronisation. Der zweite Reiz besteht vorzugsweise aus nur einem Einzelreiz, oder auch aus einer kurzen periodischen Reizfolge, die aus mindestens 2 Einzelreizen und vorzugsweise aus nicht mehr als 10 Einzelreizen besteht. Zu diesem Zweck verfügt die erfindungsgemäße Vorrichtung über Mittel zur Desynchronisation. Hierbei handelt es sich, wie weiter oben beispielhaft angegeben, um einen Computer, eine elektronische Schaltung, einen Prozessor, eine programmierbare elektronische Schaltung (FPGA) oder ein Computerprogramm, welche dazu in der Lage sind, die im Folgenden dargestellten Verfahrensschritte durchzuführen.

Die für die Desynchronisation notwendigen Stimulationsparameter werden erfindungsgemäß mit folgender Eichprozedur bestimmt.

1.) Überprüfung der Güte des Entrainments:

Eine aus k vorzugsweise identischen Reizen bestehende Reizfolge wird 1 mal, vorzugsweise 10-100 mal, appliziert. Hierbei wird n von kleinen Werten aus nach oben variiert, bis das Entrainment gut genug ist. Die Güte des Entrainments wird dabei auf folgende Weise untersucht bzw. quantifiziert:
Die Phase und die Amplitude des zu desynchronisierenden neuronalen Rhythmus wird vorzugsweise mit der Hilbert-Transformation bestimmt. Eine Alternativmethode wäre die in einem gleitenden Zeitfenster durchgeführte Anpassung des Signals des neuronalen Rhythmus an eine langsam veränderliche Sinusfunktion. Hierzu umfaßt die erfindungsgemäße Vorrichtung Mittel zur Prüfung der Güte des Entrainments. Hierbei handelt es sich, wie weiter oben beispielhaft angegeben, um einen Computer, eine elektronische Schaltung, einen Prozessor, eine programmierbare elektronische Schaltung (FPGA) oder ein Computerprogramm, welche die eben beschriebenen Schritte durchführen können. Die Wirkung des Entrainments ist es, daß nach der entrainenden Simulation der neuronale Rhythmus immer dieselbe Amplitude und vor allem immer dieselbe Phase hat, unabhängig von der Amplitude und der Phase zu Beginn der Stimulation. Um dies zu beurteilen, werden die Phase oder vorzugsweise die Phase und Amplitude durch Mittel zur Auswertung von Phase und Amplitude, in der weniger bevorzugten Ausführungsform der Vorrichtung ausschließlich durch Mittel zur Auswertung der Phase des neuronalen Rhythmus in folgender Weise ausgewertet. Hierbei handelt es sich, wie weiter oben beispielhaft angegeben, um einen Computer, eine elektronische Schaltung, einen Prozessor, eine programmierbare elektronische Schaltung (FPGA) oder ein Computerprogramm, welche die eben beschriebenen Schritte durchführen können.
Für die 1 applizierten Reizfolgen, die aus jeweils n Reizen bestehen, werden mit Mitteln zur Durchführung eines Phasen-resetting eine sogenannte Phasen-resetting-Kurve erstellt. Bei einer Phasen-resetting-Kurve handelt es sich um eine Phasenantwort-Kurve, bei der die Phase am Ende der Stimulation versus die Phase zu Beginn der Stimulation für alle m applizierten Reizfolgen aufgetragen wird. Ein perfektes Entrainment führt zu einer waagerechten Phasen-resetting-Kurve, das heißt, unabhängig von der Phase zu Beginn der Stimulation nimmt die Phase am Ende der Stimulation immer denselben Wert an.

Die Phasen-resetting-Kurve wird einerseits über ein Mittel zur Visualisierung 6, beispielsweise einen Bildschirm, dem Untersucher dargeboten. Andererseits wird die Phasen-resetting-Kurve durch einfache mathematische Operationen - wie die Standardabweichung der Werte der Phase am Ende der Stimulation oder die Güte der Anpassung einer horizontalen Gerade an die Phasen-resetting-Kurve - durch Mittel zur quantitativen Charakterisierung der Phase-resetting-Kurve, die die beispielhaft genannten Verfahrensweise durchführen, quantitativ charakterisiert. Hierbei handelt es sich, wie weiter oben beispielhaft angegeben, um einen Computer, eine elektronische Schaltung, einen Prozessor, eine programmierbare elektronische Schaltung (FPGA) oder ein Computerprogramm, welche die eben beschriebenen Schritte durchführen können.

Vorzugsweise wird die Güte des Entrainments ausschließlich visuell vom Untersucher über das Mittel zur Überprüfung der Stimulation 6 bestimmt. Die Amplitude wird in gleicher Weise mittels Amplituden-resetting-Kurven bestimmt. Hierzu verfügt die erfindungsgemäße Vorrichtung über Mittel zur Bestimmung der Amplitude und zur Durchführung eines Amplituden-resettings, die in der folgenden Weise arbeiten. Hierbei handelt es sich, wie weiter oben beispielhaft angegeben, um einen Computer, eine elektronische Schaltung, einen Prozessor, eine programmierbare elektronische Schaltung (FPGA) oder ein Computerprogramm, welche die eben beschriebenen Schritte durchführen können. In den Amplituden-resetting-Kurven, das heißt, Amplitudenantwort-Kurven, wird die Amplitude am Ende der Stimulation versus die Amplitude zu Beginn der Stimulation für alle m applizierten Reizfolgen aufgetragen. Ein perfektes Entrainment führt zu einer waagrechten Amplituden-resetting-Kurve, das heißt, unabhängig von der Amplitude zu Beginn der Stimulation nimmt die Amplitude am Ende der Stimulation immer denselben Wert an. Die Amplituden-resetting-Kurven werden wie die Phasen-resetting-Kurven quantitativ und/oder und vorzugsweise nur visuell ausgewertet. Die Anzahl der in einer Reizfolge aufeinander folgenden k Reize wird so lang erhöht, bis das Entrainment von Amplitude und Phase hinreichend gut ist.

2.) In einer alternativen und bevorzugten Ausführungsform der Erfindung kann die Güte des Entrainments in folgender Weise untersucht und quantifiziert werden. Ziel dieses alternativen Vorgehens ist es, die Güte des Entrainments nicht nur am Ende, sondern während der Applikation der gesamten Reizfolge abzuschätzen. Dies macht die Abschätzung der Güte weniger anfällig von Schwankungen der gemessenen neuronalen Dynamik, die entweder durch den Meßprozeß oder vor allem durch intrinsische neuronale Rauschkräfte bedingt sind. Hierzu wird eine aus k vorzugsweise identischen Reizen bestehende Reizfolge 1 mal vorzugsweise 10-100 mal appliziert. K wird dabei von kleinen Werten aus nach oben variiert, bis das Entrainment gut genug ist. die Güte des Entrainments wird hierbei auf folgende Weise untersucht bzw. quantifiziert:

Das Signal der über Sensor 2 gemessenen angeregten neuronalen Aktivität wird mit einem Bandpass gefiltert, der den bei der Resonanzfrequenz $f_R$ (Formel 1) gelegenen Frequenzpeak vollständig enthält, andere Frequenzpeaks, die Harmonischen, Subharmonischen oder anderen physiologischen beziehungsweise pathologischen Rhythmen entsprechen, aber nicht enthält. Mit der Hilbert-Transformation wird die Phase $\varphi_R$, das heißt, die Phase des auf diese Weise bandpaßgefilterten Signals bestimmt. Außerdem wird die Phase $\varphi_A$, das heißt die Phase der anregenden Reizfolge, bestimmt. Dies kann auf zweierlei Weise geschehen: Entweder wird eine Sinusfunktion an die Reizfolge so angepaßt, daß die Maxima der Sinusfunktion mit den Zeitpunkten, an denen die Einzelreize appliziert werden, koinzidieren. Die Phase $\varphi_A$ ist dann die Phase der angepaßten Sinusfunktion. Alternativ kann auch das Signal, welches die Reizfolge repräsentiert, also die Folge von Rechteckpulsen, mit dem zur Anregungsfrequenz $f_A$ aus Formel 1 gehörenden Bandpaß gefiltert werden. Die Phase $\varphi_A$ ist dann die mit der Hilbert-Transformation bestimmte Phase des bandpaßgefilterten Signals der Reizfolge. Der hierzu verwendete Bandpaß muß so gewählt werden, daß er den Frequenzpeak bei $f_A$ im Spektrum des Signals der Reizfolge vollständig, sonst aber keinen anderen Frequenzpeak enthält. Dann wird die *n:m*-Phasendifferenz $n\varphi_A - m\varphi_b$ zwischen der anregenden Reizfolge und der angeregten neuronalen Aktivität bestimmt. Die Stärke des Entrainments wird dann vorzugsweise mittels eines n:m-Entrainment-Index bestimmt, der wie folgt definiert ist: In dem zur Bestimmung der Güte des Entrainments verwendeten Zeitfenster wird die Verteilung der n:m-Phasendifferenz bestimmt. Dann wird die Entropie S dieser Verteilung gemäß der Formel 2

$$S = -\sum_{k=1}^{N} p_k \ln p_k \quad \text{(Formel 2)}$$

bestimmt, wobei $p_k$ die relative Häufigkeit ist, mit der sich die Werte der *n:m*-Phasendifferenz im k-ten bin befinden. Die Anzahl der bins *N* wird typischerweise gemäß Formel 3 ermittelt:

$$N = \exp\left[0.626 + 0.4\ln(M-1)\right] \qquad \text{(Formel 3)}$$

wobei *M* die Anzahl der während einer Reizfolge gemessenen Werte der *n:m*-Phasendifferenz ist.
Der *n:m*-Entrainment-Index $e_{n,m}$ berechnet sich hiermit gemäß der Formel 4

$$e_{n,m} = \frac{S_{\max} - S}{S_{\max}} \qquad \text{(Formel 4)},$$

wobei $S_{\max}$ die Entropie einer Gleichverteilung ist, das heißt, $S_{\max} = \ln N$, wobei die optimale Anzahl der zur Bestimmung der Verteilung verwendeten äquidistanten Teilintervalle (bins) durch die Formel 3 gegeben ist. Durch die mittels Formel 4 erzielte Normalisierung ist $0 \leq e_{n,m} \leq 1$ stets erfüllt. $e_{n,m} = 0$ bedeutet, daß kein Entrainment vorliegt, während $e_{n,m} = 1$ einem perfekten Entrainment entspricht. Je größer $e_{n,m}$ ist, desto besser ist das Entrainment ausgebildet.
$e_{n,m}$ wird jeweils für die 1 applizierten Reizfolgen berechnet. Daraus wird dann der Mittelwert

$$E_{n,m} = \frac{1}{l}\sum_{j=1}^{l} e_{n,m}^{(j)} \qquad \text{(Formel 5)}$$

berechnet, wobei $e_{n,m}^{(j)}$ der j-ten Reizfolge ist. Es gilt $0 \leq E_{n,m} \leq 1$. Die Anzahl der in einer Reizfolge aufeinanderfolgenden k Reize wird solange erhöht, bis das Entrainment hinreichend gut, das heißt bis $E_{n,m}$ hinreichend nahe bei 1 ist.

2.) Ermittlung der vulnerablen Phase:

Die vulnerable Phase hängt von der Intensität und der Dauer des sensorischen Reizes ab. Vorzugsweise wird im Rahmen der Eichprozedur die Dauer des sensorischen Reizes konstant gehalten, während die Intensität und die vulnerable Phase wie unten beschrieben so variiert werden, daß der desynchronisierende Effekt der Reizung maximiert wird.
Die Ermittlung der vulnerablen Phase erfolgt über Mittel zur Bestimmung der vulnerablen Phase. Hierbei handelt es sich, wie weiter oben beispielhaft angegeben, um einen Computer, eine elektronische Schaltung, einen Prozessor, eine programmierbare elektronische Schaltung (FPGA) oder ein Computerprogramm, welche zur Durchführung der im Folgenden beschriebenen Schritte befähigt sind. Hierbei kann die erfindungsgemäße Vorrichtung auf zwei verschiedenen Weisen vorgehen:

A) Der zeitliche Abstand zwischen dem letzten Reiz der entrainenden Reizfolge und dem desynchronisierenden Reiz einerseits und die Intensität des desynchronisierenden Reizes wird durch Mittel zum Variieren des zeitlichen Abstandes zwischen dem letzten Reiz des Entrainments und des desynchronisierenden Reizes zwischen vorzugsweise 0 und 2 Periodenlängen der mittleren Frequenz des zum pathologischen Rhythmus gehörenden Frequenzbandes systematisch, vorzugsweise in kleinen äquidistanten Schritten variiert. Bei den hierzu verwendeten Mitteln handelt es sich, wie weiter oben beispielhaft angegeben, um einen Computer, eine elektronische Schaltung, einen Prozessor, eine programmierbare elektronische Schaltung (FPGA) oder ein Computerprogramm. Diese Variation des zeitlichen Abstandes wird systematisch für verschiedene Werte der Intensität mittels eines Mittels zur Variation der Intensität durchgeführt. Vorzugsweise wird dabei die Intensität in kleinen äquidistanten Schritten erhöht und für jeden Wert der Intensität wird der zeitliche Abstand wie oben beschrieben zwischen 0 und 2 Periodenlängen bestimmt. Die Variation des zeitlichen Abstandes und der Intensität wird vorzugsweise durch die Steuereinheit 4 vorgenommen. Die optimalen Werte für Intensität des sensorischen Reizes und dem Zeitabstand zwischen dem letzten Reiz des Entrainments und dem desynchronisierenden Reiz sind die Werte, bei denen die stärkste Desynchronisation auftritt, das heißt, bei der die Amplitude des zu desynchronisierenden Rhythmus nach Stimulation am geringsten ist. Die Amplitude wird hierbei vorzugsweise durch Bandpaßfilterung mit nachfolgender Hilbert-Transformation bestimmt. Alternativ kann die Amplitude auch entweder durch die Anpassung einer langsam veränderlichen Sinusfunktion an das bandpaßgefilterte Signal von Sensor 2 in einem Zeitfenster nach Stimulation oder über die über das Frequenzband integrierte Amplitude des Powerspektrums des über Sensor 2 in einem Zeitfenster nach Stimulation gemessenen Signals bestimmt werden. B) Der zeitliche Abstand wird wie unter A) variiert. Im Unterschied zu A) wird nun aber die Intensität nicht in äquidistanten Schritten erhöht, sondern in folgenderweise systematisch variiert: Hierbei werden Phasen-resetting-Kurven verwendet, mit denen die Auswirkung des desynchronisierenden Reizes auf die Phasendynamik der zu desynchronisierenden neuronalen Aktivität untersucht wird. Die Phase wird dabei vorzugsweise mittels Bandpaßfilterung und nachfolgender Hilbert-Transformation des über Sensor 2 gemessenen Signals bestimmt. Alternativ zur Verwendung der Hilbert-Transformation kann auch in einem gleitenden Zeitfenster eine langsam veränderliche Sinusfunktion an das bandpaßgefilterte Signal von Sensor 2 angepaßt werden. Die Grenzen des Bandpasses sind dabei die Grenzen des Frequenzbandes des pathologischen neuronalen Rhythmus, die anfänglich bestimmt wurden. Bei den Phasen-resetting-Kurven wird $\varphi_e$ über $\varphi_b$ über ein Mittel zum Auftragen von $\varphi_e$, der Phase der neuronalen Aktivität nach Stimulation, über $\varphi_b$, die Phase der neuronalen Aktivität zu Beginn der Stimulation, aufgetragen, welches ein Mittel zum Untersuchen der Auswirkung des desynchronisierenden Reizes auf die Phasendynamik der zu desynchronisierenden neuronalen Aktivität darstellt. Hierbei handelt es sich, wie weiter oben beispielhaft angegeben, um einen Computer, eine elektronische Schaltung, einen Prozessor, eine programmierbare elektronische Schaltung (FPGA) oder ein Computerprogramm. $\varphi_e$ ist dabei die Phase der neuronalen Aktivität, die entweder unmittelbar nach Stimulation oder mit einer konstanten Zeitverzögerung nach Stimulation bestimmt wird. Diese Zeitverzögerung soll dabei vorzugsweise kleiner als eine Periodenlänge des zu desynchronisierenden neuronalen Rhythmus oder am besten gleich null sein. Da die Periodenlänge des neuronalen Rhythmus zeitlich variiert, ist oben mit Periodenlänge die zeitlich gemittelte Periodenlänge gemeint. $\varphi_b$ ist die Phase der neuronalen Aktivität, die entweder unmittelbar zum Zeitpunkt des Stimulationsbeginns oder mit einer konstanten Zeitverzögerung vor Stimulationsbeginn bestimmt wird. Die Zeitverzögerung sollte analog zur Bestimmung zu $\varphi_e$ möglichst klein oder am besten gleich null sein. Die Zeitverzögerung bei der Bestimmung von $\varphi_e$ oder $\varphi_e$ soll deswegen möglichst gering sein, damit die unumgänglichen zeitlichen Variationen der Periodenlänge, die Güte der Auswertung möglichst nicht beeinträchtigen. Ist die gewählte Intensität des desynchronisierenden sensorischen Reizes zu gering, so hat die Phasen-resetting-Kurve typischerweise eine mittlere Steigung von 1. Ist hingegen die Intensität zu groß, so hat die Phasen-resetting-Kurve typischerweise eine mittlere Steigung von null. Der optimale Intensitätswert und der optimale Wert für die Verzögerung zwischen dem letzten entrainenden Puls und dem desynchronisierenden Puls finden sich genau an der Stelle der Phasen-resetting-Kurve, bei der der Übergang von einer mittleren Steigung 1 zu einer mittleren Steigung 0 auftritt.

Dies ist in Figur 5 dargestellt. Figur 5a bis 5f zeigen jeweils eine Phasen-resetting-Kurve, wobei in den einzelnen Teilabbildungen die Intensität des sensorischen Reizes konstant ist, aber zwischen den Teilabbildungen verschieden ist, und zwar von Figur 5a nach Figur 5f von kleinen nach großen Werten hin anwächst. Die optimalen Stimulationsparameter finden sich beim Übergang von Figur 5c nach 5e an der mit dem Pfeil markierten Stelle, das heißt, (i) der Mittelwert der zu Figur 5c und 5d gehörigen Intensitäten ist für die gewählte Reizdauer die optimale, am stärksten desynchronisierende Intensität und (ii) die in Figur 5d mit dem Pfeil am Umschlagpunkt markierte Phase $\varphi_b$ ist der Phasenwert, der dem für die gewählte Reizdauer optimale, am stärksten desynchronisierende Zeitabstand zwischen dem letzten Reiz des Entrainments und dem desynchronisierenden Reiz entspricht. Dieser Zeitabstand kann entweder in einer absoluten Zeit oder - analog dazu - wie in Figur 5 dargestellt als Phase der neuronalen Aktivität angegeben werden. Bei den Phasen-resetting-Kurven kann dementsprechend als x-Achse äquivalent zu $\varphi_b$ auch der absolute Zeitabstand zwischen dem letzten Reiz des Entrainments und dem desynchronisierenden Reiz angegeben werden. Sind die experimentellen Daten stark verrauscht, so werden bei der Erstellung einer Phasen-resetting-Kurve jeweils für ein Wertepaar bestehend aus Intensität und $\varphi_b$ mehrere Messungen durchgeführt und der mittlere Wert von $\varphi_e$ verwendet.

Die Steuereinheit 4 kontrolliert auf zwei verschiedene Weisen die sensorische Stimulation. Die bedarfsgesteuerte Desynchronisation kann entweder repetitiv oder anhaltend durchgeführt werden. Bei beiden Funktionsweisen wird dabei für die effektive Desynchronisation ein Entrainment verwendet. Die Frequenz des Entrainments, das heißt die Rate der entrainenden Folge sensorischer Reize, wird im vorher durchgeführten Frequenzscan ermittelt. Dabei wird bestimmt, bei welcher Anregungsfrequenz $f_A$ die Amplitude des pathologischen Rhythmus maximal ist. Ist die Anregungsfrequenz $f_A$ identifiziert, bzw. sind mehrere Anregungsfrequenzen identifiziert, so kann mit der Desynchronisation begonnen werden. Falls mehrere Anregungsfrequenzen gefunden werden, so wird für die Desynchronisation diejenige verwandt, die den stärksten Entrainmenteffekt, das heißt die stärkste Anregung der Amplitude, hervorruft.

a) Repetitive Anwendung:

Bei der repetitiven Anwendung wird dieselbe desynchronisierende Reizfolge repetitiv dargeboten. In den Pausen zwischen diesen desynchronisierenden Reizfolgen erfolgt keine Reizdarbietung.

Der Patient wird vor Beginn der bedarfsgesteuerten Desynchronisation durch einen Untersucher bzw. durch das Gerät instruiert. Das heißt, dem Patienten wird entweder vom Untersucher gesagt, wie er die repetitiv dargebotenen Reizfolgen verarbeiten soll, oder das Gerät signalisiert dies dem Patienten, zum Beispiel durch visuelle oder auditorische Anweisungen: Der Patient liest oder hört, was er zu tun hat.

Zum Beispiel muß der Patient bei visueller Stimulation in den repetitiv dargebotenen visuellen Reizmustern nach bestimmten Objekten bzw. Einzelmustern, zum Beispiel Kanisza-Figuren, suchen, diese zählen beziehungsweise diese miteinander vergleichen. Der Untersucher kontrolliert mit dem Mittel zur Überprüfung der Stimulation 6 dabei vorzugsweise die Wirkung der Stimulation auf die Hirnaktivität und die Informationsverarbeitung des Patienten, welche über den Taster 5 rückgemeldet wird. Der Patient muß zum Beispiel jedesmal, wenn er bestimmte Teilmuster erkannt hat, den Taster 5 drücken. Auf diese Weise kontrolliert der Untersucher, ob die Erkennung der dargebotenen sensorischen Reize durch die Dämpfung bzw. Unterdrückung des pathologischen Rhythmus verbessert bzw. ermöglicht wird. Bleibt die Reaktion des Patienten mindestens einmal aus, so wird von der Steuereinheit 4 ein geeignetes Signal an das Mittel zur Überprüfung der Stimulation 6 und damit an den Untersucher weitergeleitet. Dieses Signal dient dem Zweck, dem Untersucher mitzuteilen, daß der Patient nicht willens oder nicht in der Lage ist, die sensorischen Reize gemäß der vorgegebenen Aufgabe zu verarbeiten.

Die Steuereinheit 4 steuert die Darbietung der sensorischen Reize in folgender Weise:

Eine entrainende, periodische Abfolge von sensorischen Reizen mit der optimalen Anregungsfrequenz $f_A$ wird appliziert. Die hierzu verwendeten sensorischen Reize können aber müssen nicht identisch sein. Vorzugsweise sind die verwendeten sensorischen Reize bezüglich der folgenden Parameter identisch, um ein zügiges Entrainment zu realisieren:

(i) Sie sind von derselben Qualität, das heißt, es handelt sich zum Beispiel jeweils um dasselbe visuelle Muster.
(ii) Sie haben dieselbe Intensität, das heißt, zum Beispiel dieselbe Leucht- oder Lautstärke.
(iii) Sie haben denselben Kontrast, das heißt zum Beispiel, bei visuellen Reizen denselben Hell-Dunkel-Kontrast.
(iv) Sie haben dieselbe Dauer.

Mit einer konstanten Verzögerung erfolgt daraufhin die Applikation des desynchronisierenden Reizes in der vulnerablen Phasenlage des pathologischen Rhythmus. Der desynchronisierende sensorische Reiz ist vorzugsweise von derselben Modalität, das heißt, wenn die entrainenden Reize visuelle Reize sind, so ist auch der desynchronisierende Reiz ein visueller Reiz und zum Beispiel kein auditorischer Reiz.

Der desynchronisierende Reiz kann aber muß nicht von derselben Qualität sein, wie der entrainende. Vorzugsweise ist er von derselben Qualität, das heißt er hat zum Beispiel das gleiche visuelle Muster. Der desynchronisierende Reiz unterscheidet sich aber vorzugsweise von den Reizen der entrainenden Reizfolge durch seine Dauer und/oder seine Intensität und/oder seinen Kontrast.

Sobald der desynchronisierende Reiz appliziert ist, wird vorübergehend kein Reiz präsentiert. Im Anschluß an die Reizdarbietung muß der Patient vorzugsweise über den Taster 5 rückmelden, ob er die gestellte Aufgabe, zum Beispiel das Auffinden spezieller Objekte oder visueller Muster, zu lösen imstande war. Es vergeht nach einem solchen desynchronisierenden Reiz eine Pause, deren Dauer in einem vorgegebenen Intervall statistisch verteilt - vorzugsweise gleichverteilt - ist. Während dieser Pause wird keine Reizung durchgeführt. Nach dieser Pause erfolgt die nächste Reizung mit demselben zusammengesetzten desynchronisierenden Stimulus, der aus einer entrainenden Reizfolge und einem desynchronisierenden Einzelreiz besteht.

Im Rahmen der repetitiven Anwendung kontrolliert die Steuereinheit 4, ob die Desynchronisation der pathologisch aktiven Neuronenpopulation effektiv ist, das heißt, ob die Dämpfung des pathologischen Rhythmus stark genug ist. Falls dies der Fall ist, wird fortlaufend, repetitiv stimuliert. Falls die Dämpfung des pathologischen Rhythmus mindestens einmal ungenügend ist, muß neu mit der oben beschriebenen Eichprozedur geeicht werden.

Fig. 2 zeigt eine Anregung mit der Resonanzfrequenz, nach der in der vulnerablen Phase ein desynchronisierender Puls erfolgt. Hierbei ist auf der x-Achse die Zeit und auf der y-Achse die Intensität der sensorischen Reize aufgetragen.

b) Anhaltende Anwendung:

Anders als bei der repetitiven Anwendung a) wird bei der anhaltenden Anwendung permanent sensorisch stimuliert. Immer wenn ein Schwellenwert der wie oben beschrieben bestimmten Amplitude der zu desynchronisierenden neuronalen Aktivität überschritten wird, wird eine Desynchronisation vogenommen. Hierzu wird eine entrainendene Pulsfolge gefolgt von mindestens einem Einzelreiz appliziert (Fig. 2). In den Zeiten zwischen den Desynchronisationen erfolgt eine anhaltende sensorische Stimulation. Hierbei gibt es zwei Möglichkeiten:

I) In den Zeiten zwischen den Desynchronisationen wird mit einer periodischen Abfolge von sensorischen Reizen stimuliert. Diese Abfolge besteht aus identischen Einzelreizen, die mit einer Frequenz dargeboten werden, die von der Resonanzfrequenz hinreichend verschieden ist, so daß es zu keiner Resonanz kommt.

II) In den Zeiten zwischen den Desynchronisationen wird mit einer stochastischen Abfolge von sensorischen Reizen stimuliert. Die Einzelreize dieser Abfolge bestehen aus identischen visuellen oder auditorischen Mustern, bei denen folgende Parameter von Reiz zu Reiz statistisch variiert werden: Bei visuellen Reizen kann der Kontrast und/oder die Helligkeit variiert werden. Bei auditorischen Reizen kann die Lautstärke variiert werden. Zudem kann die Pause zwischen den Einzelreizen und die Dauer der Einzelreize statistisch variiert werden. Bei der statistischen Variation werden dabei die entsprechenden Parameter in bei physiologischen Experimenten gebräuchlichen Grenzen normal- oder gleichverteilt variiert.

Zweck der oben unter I und II beschriebenen Stimulation ist es, 1. dem Patienten dauerhaft den von ihm zu verarbeitenden sensorischen Stimulus darzubieten, so daß der Patient anhaltend die an ihn gestellte Aufgabe, zum Beispiel das Auffinden visueller Teilmuster, ausführen kann, und 2. hierbei eine Resonanz des pathologischen Rhythmus zu verhindern.

Figur 3a zeigt einen beispielhaften Verlauf für die mit dem Mittel zur Erzeugung der sensorischen Reize 1 erzeugten zeitlichen Muster des sensorischen Reizes, wobei Variante I, das heißt eine periodische Stimulation zwischen den Desynchonisationen, verwendet wird. In Figur 3b sind die zugehörigen Aktivitätsmuster der erkrankten Hirnregion angegeben. In Figur 3a und 3b ist die x-Achse jeweils die Zeitachse. In Figur 3a ist auf der y-Achse die Intensität der Reize aufgetragen. In Figur 3b ist die in einem gleitenden Zeitfenster zeitlich gemittelte Amplitude der zu desynchronisierenden neuronalen Aktivität aufgetragen.

In der Figur 3a und 3b sind die Zeitbereiche $T_1$ und $T_1'$ , $T_2$ und $T_2'$, $T_3$ und $T_3'$ , $T_4$ und $T_4'$ sowie $T_5$ und $T_5'$ identisch. Im Zeitbereich $T_1$ beziehungsweise $T_1'$ ist die Amplitude des pathologischen Rhythmus durch Resonanz maximal. Im Zeitbereich $T_2$ bzw. $T_2'$ wird in der vulnerablen Phase ein desynchronisierender sensorischer Reiz gesetzt, der die pathologische Aktivität entweder vollkommen unterdrückt oder zumindest in ihrer Intensität reduziert. Dies führt zu einem Abklingen der Amplitude in Fig.3b im Zeitabschnitt $T_2'$.

Wie oben unter I beschrieben wird in dem Zeitbereich $T_3$ eine periodische Reizfolge appliziert, deren Frequenz hinreichend von der im Zeitbereich $T_1$ verwendeten Resonanzfrequenz verschieden ist. Hierdurch wird im Zeitbereich $T_3'$ bewirkt, daß sich trotz sensorischer Stimulation der pathologische Rhythmus nur langsam wieder aufschaukelt. In der Fallgestaltung II wird im Zeitbereich $T_3$ statt der periodischen Reizfolge eine stochastische Reizfolge verwendet. Mit dieser Maßnahme wird der pathologische Rhythmus so lange wie möglich unterdrückt. In Figur 3b ist diese Phase durch den Abschnitt $T_3'$ charakterisiert, in dem die Kurve der zu unterdrückenden Hirnaktivität minimale Werte annimmt. Sobald im Zeitbereich $T_3'$ die Hirnaktivität wieder einen Schwellenwert überschritten hat, tritt der Bedarfsfall der Desynchronisation auf, so daß im Zeitbereich $T_4$ eine erneute Desynchronisation durchgeführt wird. Dabei erfolgt im Zeitbereich $T_4$ dasselbe Entrainment wie im Zeitbereich $T_1$. Nach dem Entrainment wird im Zeitbereich $T_5$ ein desynchronisierender Reiz wie in Zeitbereich $T_2$ appliziert. Hierbei registriert der Sensor 2 die zunehmende Aktivität der kranken Hirnregion, und gibt die Signale an die Steuereinheit 4 weiter, die die nächste Desynchronisation auslöst. Im Anschluß an die im Zeitbereich $T_5$ erfolgte Desynchronisation wird aufs neue wie im Zeitbereich $T_3$ eine periodische Reizfolge appliziert, deren Frequenz hinreichend von der Resonanzfrequenz unterschiedlich ist. Die entspricht der oben beschriebenen Fallgestaltung I. Alternativ hierzu kann auch gemäß oben beschriebener Fallgestaltung II eine stochastische Reizfolge verwendet werden.

Die Erfindung umfaßt ein Computerprogramm mit Programmcode-Mitteln zur Steuerung einer Vorrichtung, die wenigstens einen der vorgehenden Verfahrensschritte oder eine beliebige Kombination wenigstens von zwei der in der Beschreibung angegebenen Verfahrensschritte durchgeführt, wenn das Programm auf einem Computer ausgeführt wird.

Weiterhin umfaßt die Erfindung ein Computerprogrammprodukt mit Programmcodemitteln, die auf einem computerlesbaren Datenträger gespeichert sind um das Verfahren, welches dem Computerprogramm zu Grunde liegt durchzuführen. Dieses Computerprorammprodukt kann beispielsweise eine Diskette sein.

Weiterhin umfaßt die Erfindung eine elektronische Schaltung, die befähigt ist, die Anweisungen des Computerprogramms oder des Computerprogrammprodukts auszuführen.

**Patentansprüche**

1. Vorrichtung zur bedarfsgesteuerten Desynchronisation einer pathologisch rhythmisch aktiven Neuronenpopulation eines Patienten, umfassend:

   - eine Steuereinheit (4),
   - einen Stimulator (1) zur Erzeugung visueller und/oder auditorischer und/oder taktiler Reize, die die Form von Pulsen haben und vom Patienten aufgenommen werden, und
   - mindestens ein Mittel zur Erfassung von Hirnaktivitäten (2) des Patienten, wobei der Stimulator (1) und das mindestens eine Mittel zur Erfassung von Hirnaktivitäten (2) mit der Steuereinheit (4) in Verbindung stehen,
   **dadurch gekennzeichnet,**
   - **daß** die Steuereinheit (4)

      - ein erstes Mittel, welches den Stimulator (1) derart ansteuert, dass der Stimulator (1) eine Pulsfolge erzeugt, wobei die Frequenz, mit der die Pulse der Pulsfolge erzeugt werden, variiert wird und wobei das erste Mittel anhand der von dem Mittel zur Erfassung der Hirnaktivitäten (2) während der Erzeugung der Pulsfolge aufgenommenen Hirnaktivität ermittelt, bei welcher ersten Frequenz der erzeugten Pulse neuronale Aktivität des Patienten angeregt wird, und
      - ein zweites Mittel, welches den Stimulator (1) derart ansteuert, dass der Stimulator (1) eine periodische Pulsfolge, deren Pulse mit der ersten Frequenz- wiederholt werden, und einen anschließenden Puls erzeugt, wobei die Erzeugung der periodischen Pulsfolge und des anschließenden Pulses mehrfach durchgeführt wird und dabei der zeitliche Abstand zwischen der periodischen Pulsfolge und dem anschließenden Puls und die Intensität des anschließenden Pulses variiert werden, wobei das zweite Mittel anhand der von dem Mittel zur Erfassung der Hirnaktivitäten (2) während der mehrfachen Erzeugung der periodischen Pulsfolge und des anschließenden Pulses aufgenommenen Hirnaktivität ermittelt, bei welchem ersten zeitlichen Abstand zwischen der periodischen Pulsfolge und dem anschließenden Puls und bei welcher ersten Intensität des anschließenden Pulses die pathologisch rhythmisch aktive Neuronenpopulation am stärksten desynchronisiert wird,

   umfaßt, und
   - **daß** die Steuereinheit (4) derart ausgestaltet ist, daß die Steuereinheit (4) nach der Ermittlung der ersten Frequenz, des ersten zeitlichen Abstands und der ersten Intensität den Stimulator (1) derart ansteuert, dass der Stimulator (1) eine periodische Pulsfolge, deren Pulse mit der ersten Frequenz wiederholt werden, und einen anschließenden Puls erzeugt, wobei der anschließende Puls mit dem ersten zeitlichen Abstand auf die periodische Pulsfolge folgt und der anschließende Puls die erste Intensität aufweist.

2. Vorrichtung nach Anspruch 1,
   **dadurch gekennzeichnet,**

   - **daß** der Stimulator (1) mindestens eine Komponente aus der Gruppe bestehend aus einem Bildschirm, einer Shutterbrille, einem Lautsprecher, einem Kopfhörer, einem Druckgeber und einem zeitlich modulierten Laser ist.

3. Vorrichtung nach Anspruch 1 oder 2,
   **dadurch gekennzeichnet,**

   - **daß** das Mittel zur Erfassung von Hirnaktivitäten (2) mindestens eine Komponente aus der Gruppe bestehend aus einer Skalp-EEG-Elektrode und einem MEG-Sensor ist.

4. Vorrichtung nach einem der Ansprüche 1 bis 3,
   **dadurch gekennzeichnet,**

   - **daß** das Mittel zur Erfassung von Hirnaktivitäten (2) über einen Trennverstärker (3) mit der Steuereinheit (4) verbunden ist.

**5.** Vorrichtung nach einem der Ansprüche 1 bis 4,
**dadurch gekennzeichnet,**

- **daß** sie ein Mittel zur Rückmeldung einer Reaktion des Patienten (5) umfaßt, welches an die Steuereinheit (4) angeschlossen ist.

**6.** Vorrichtung nach einem der Ansprüche 1 bis 5,
**dadurch gekennzeichnet,**

- **daß** das erste Mittel den Stimulator (1) derart ansteuert, dass die Frequenz der von dem Stimulator (1) erzeugten Pulsfolge zwischen 1 und 100 Hz variiert wird.

**7.** Vorrichtung nach einem der Ansprüche 1 bis 6,
**dadurch gekennzeichnet,**

- **daß** der anschließende Puls ein Einzelpuls oder der erste Puls einer periodischen Pulsfolge mit 2 bis 10 Pulsen ist.


**Claims**

**1.** A device for demand-controlled desynchronization of a pathological, rhythmically active neuron population of a patient, comprising:

- a controller (4)
- a stimulator (1) for generating visual and/or auditory and/or tactile stimuli having the form of pulses received by the patient, and
- at least one means for detecting activities of the brain (2) of the patient, the stimulator (1) and the at least one means for detecting activities of the brain (2) being connected to the controller (4)
**characterized in**
- **that** the controller (4) comprises
- a first means activating the stimulator (1) such that the stimulator (1) generates a train of pulses, the frequency with which the pulses of the train of pulses is generated being varied and the first means detecting from the cerebral activity recorded by the means for detecting the activities of the brain (2) during generation of the train of pulses at which first frequency of the generated pulses neuronal activity of the patient is triggered, and
- a second means activating the stimulator (1) such that the stimulator (1) generates a periodic train of pulses, the pulses of which are repeated with the first frequency, and a subsequent pulse, generating the periodic train of pulses and the subsequent pulse being performed multiply in thereby varying the time spacing between the periodic train of pulses and the subsequent pulse as well as varying the intensity of the subsequent pulse, the second means detecting from the cerebral activity recorded by the means for detecting the activities of the brain (2) during multiple generation of the periodic train of pulses and the subsequent pulse at which first time spacing between the periodic train of pulses and the subsequent pulse and at which first intensity of the subsequent pulse the pathological, rhythmically active neuron population is desynchronized the strongestly and
- **that** the controller (4) is configured such that the controller (4) after detection of the first frequency, the first time spacing and the first intensity activates the stimulator (1) such that the stimulator (1) generates a periodic train of pulses, the pulses of which are repeated with the first frequency, and a subsequent pulse, the subsequent pulse following the periodic train of pulses with the first time spacing and the subsequent pulse comprising the first intensity.

**2.** The device as set forth in claim 1,
**characterized in that**

- the stimulator (1) is at least one component of the group comprising a screen, shutter glasses, loudspeaker, headphones, pressure transmitter and a time-modulated laser.

**3.** The device as set forth in claim 1 or 2,
**characterized in that**

- the means for detecting activities of the brain (2) is at least one component of the group comprising a scalp EEG electrode and a MEG sensor.

4. The device as set forth in any of the claims 1 to 3,
   **characterized in that**

   - the means for detecting activities of the brain (2) is connected to the controller (4) via a buffer amplifier (3).

5. The device as set forth in any of the claims 1 to 4,
   **characterized in that**

   - it comprises, connected to the controller (4), a means for feeding back a reaction of the patient (5).

6. The device as set forth in any of the claims 1 to 5,
   **characterized in that**

   - the first means activates the stimulator (1) such that the frequency of the train of pulses generated by the stimulator (1) is varied between 1 and 100 Hz.

7. The device as set forth in any of the claims 1 to 6,
   **characterized in that**

   - the subsequent pulse is a single pulse or the first pulse of a periodic train of 2 to 10 pulses.

**Revendications**

1. Dispositif pour la désynchronisation commandée selon les besoins d'une population de neurones, active pathologiquement et rythmiquement, d'un patient, comprenant :

   - une unité de commande (4),
   - un stimulateur (1) pour générer des excitations visuelles et/ou auditoires et/ou tactiles qui ont la forme d'impulsions et qui sont réceptionnées par le patient, et
   - au moins un moyen destiné à saisir des activités du cerveau (2) du patient, dans lequel le stimulateur (1) et le au moins un moyen pour saisir des activités du cerveau (2) sont en liaison avec l'unité de commande (4),
   **caractérisé**
   - **en ce que** l'unité de commande (4) comporte :

     - un premier moyen qui commande le stimulateur (1) de telle sorte que le stimulateur (1) génère une séquence d'impulsions, dans lequel la fréquence, avec laquelle les impulsions de la séquence d'impulsions sont générées, est variée et dans lequel le premier moyen détermine à quelle première fréquence des impulsions générées une activité neuronale du patient est excitée, à l'aide de l'activité du cerveau enregistrée par le moyen destiné à la saisie des activités du cerveau (2) pendant la génération de la séquence d'impulsions, et
     - un second moyen qui commande le stimulateur (1) de telle sorte que le stimulateur (1) génère une séquence d'impulsions périodique dont les impulsions sont répétées à la première fréquence, et une impulsion s'en suivant, dans lequel la génération de la séquence d'impulsions périodique et de l'impulsion s'en suivant est exécutée plusieurs fois et dans lequel la distance temporelle entre la séquence d'impulsions périodique et l'impulsion s'en suivant et l'intensité de l'impulsion s'en suivant est alors variée, dans lequel le second moyen détermine l'activité du cerveau enregistrée à l'aide de l'activité du cerveau enregistrée par le moyen destiné à la saisie des activités du cerveau (2) pendant la génération multiple de la séquence d'impulsions périodique et de l'impulsion s'en suivant, et détermine, pour quelle première distance temporelle entre la séquence d'impulsions périodique et l'impulsion s'en suivant et pour quelle première intensité de l'impulsion s'en suivant, la population de neurones, active pathologiquement et rythmiquement, est désynchronisée le plus fortement,

     et
     - **en ce que** l'unité de commande (4) est accomplie de telle sorte que - après la détermination de la première fréquence, de la première distance temporelle et de la première intensité - l'unité de commande (4) commande le stimulateur (1) de telle sorte que le stimulateur (1) génère une séquence d'impulsions périodique dont les impulsions sont répétées à la première fréquence et une impulsion s'en suivant, sachant que l'impulsion s'en suivant suit la séquence d'impulsions périodique avec la première distance temporelle et que l'impulsion s'en suivant présente la première intensité.

2. Dispositif selon la revendication 1,
   **caractérisé en ce que** :

   - le stimulateur (1) est au moins un composant parmi le groupe constitué d'un écran, d'une lunette obturatrice, d'un haut-parleur, d'un casque d'écouteurs, d'un capteur de pression et d'un laser à modulation temporelle.

3. Dispositif selon l'une ou l'autre des revendications 1 et 2,
   **caractérisé en ce que** :

   - le moyen destiné à la saisie des activités du cerveau (2) est au moins un composant parmi le groupe constitué d'une électrode d'électroencéphalogramme de scalp et d'un capteur de magnétoencéphalographie (MEG).

4. Dispositif selon l'une des revendications 1 à 3,
   **caractérisé en ce que** :

   - le moyen destiné à la saisie des activités du cerveau (2) est relié à l'unité de commande (4) par l'intermédiaire d'un amplificateur de séparation (3).

5. Dispositif selon l'une des revendications 1 à 4,
   **caractérisé en ce :**

   - **qu'**il comporte un moyen pour la signalisation en retour d'une réaction du patient (5), lequel est connecté à l'unité de commande (4).

6. Dispositif selon l'une des revendications 1 à 5,
   **caractérisé en ce que** .

   - le premier moyen commande le stimulateur (1) de telle sorte que la fréquence de la séquence d'impulsions générée par le stimulateur (1) est variée entre 1 et 100 Hz.

7. Dispositif selon l'une des revendications 1 à 6,
   **caractérisé en ce que** :

   - l'impulsion s'en suivant est une impulsion individuelle ou est la première impulsion d'une séquence d'impulsions périodique comportant 2 à 10 impulsions.

Fig.1

Fig. 2

EP 1 524 935 B1

T₁ T₂ T₃ T₄ T₅

Fig. 3a

T'₁ T'₂ T'₃ T'₄ T'₅

Fig. 3b

EP 1 524 935 B1

Fig. 4a

Fig. 4b

Fig. 5a

Fig. 5b

Fig. 5c

Fig. 5d

Fig. 5e

Fig. 5f

EP 1 524 935 B1

Fig. 6

**EP 1 524 935 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4201224 A